# DEMANDE DE BREVET EUROPEEN

(11) **EP 3 150 697 A1**
(43) Date de publication de la demande: **05.04.2017**
(21) Numéro de dépôt: 16191226.6
(22) Date de dépôt: 28.09.2016
(51) Int. Cl.: C12N 1/12, B01D 53/84, C02F 3/32, C02F 3/34, C10B 53/00

(54) **PROCEDE DE TRAITEMENT D'EFFLUENTS LIQUIDES ET DE PRODUCTION DE MICRO-ALGUES, COMPRENANT UNE ETAPE DE PYROLYSE**

(30) Priorité: 29.09.2015 FR 1559150
(71) Demandeur: COMMISSARIAT À L'ÉNERGIE ATOMIQUE ET AUX ÉNERGIES ALTERNATIVES, 75015 Paris (FR)
(72) Inventeur: DELRUE, Florian, 84120 PERTUIS (FR); DUPONT, Capucine, 38000 GRENOBLE (FR)
(74) Mandataire: Nony

(57) **Abrégé**

L'invention concerne un procédé de production de micro-algues et de traitement d'au moins un effluent liquide, comportant les étapes suivantes:
a/ culture de micro-algues dans une zone de culture dans laquelle on fait circuler le ou les effluents liquides;
b/ pyrolyse de la biomasse séchée obtenue à partir des micro-algues cultivées selon l'étape a/ ;
c/ mise en contact du char obtenu par la pyrolyse selon l'étape b/ avec le ou les effluents liquides.

## Description

### Domaine technique

La présente invention concerne un procédé à la fois de traitement d'effluents liquides et de production de micro-algues, comprenant une étape de pyrolyse de la biomasse séchée obtenue à partir de micro-algues cultivées.

L'invention vise en premier lieu à éliminer les espèces toxiques présentes dans les effluents liquides qui dans la présente invention, constituent le milieu de culture des micro-algues.

Les effluents liquides visés par l'invention sont principalement ceux d'origine urbaine, industrielle ou agricole.

L'invention permet également de rendre économiquement plus viables les procédés de production de micro-algues existantes.

Par « micro-algues », on entend le sens usuel, à savoir des algues, i.e. des organismes photosynthétiques, de taille microscopique, typiquement comprise entre 1 et 10 µm. Ainsi, dans le cadre de l'invention, tous micro-organismes photosynthétiques, dont les cyanobactéries peuvent être considérées comme des micro-algues dans le contexte de l'invention.

### Etat de la technique

A l'état naturel, les micro-algues (micro-organisme photosynthétique eucaryote ou procaryote d'une des familles suivantes : Chlorophycées, Diatomées, Coccolithophyceae, Chrysophycées, Rhodophycées, Trebouxiophyceae, Euglénophycées ou cyanobactéries), sont des organismes photosynthétiques unicellulaires, principaux composants du phytoplancton, qui peuplent les océans et cours d'eau depuis plus de trois milliards et demi d'années.

La culture des micro-algues à l'échelle industrielle connaît depuis peu un fort engouement du fait des nombreux champs d'application parmi lesquelles on peut citer, dans une échelle de valorisation décroissante, la pharmaceutique et la cosmétique, l'alimentation humaine, l'alimentation animale, les fertilisants, les matériaux biobasés, la biorémédiation et les biocarburants (liquides ou gazeux).

Les trois espèces de micro-algues actuellement les plus cultivées sont, par ordre de quantité décroissante, la cyanobactérie Arthrospira plus connue sous l'appellation de « spiruline », qui représente environ 50% de la production mondiale, suivie par les microalgues vertes Chlorella, Dunaliella, Haematococcus, Nannochloropsis et la diatomée Odontella.

Pour la culture de micro-algues, on distingue notamment deux grandes familles de systèmes de culture [1] :
- les systèmes d'architecture de type ouverte, également connus sous la dénomination « Raceway », lorsqu'ils sont optimisés (agitation par roues à aubes et forme ovale du bassin typique), qui sont constitués par des bassins ou lagunes à ciel ouvert, c'est-à-dire à l'air libre. Ils ont comme avantage principal de générer des coûts de production relativement faibles. Leurs inconvénients majeurs résident dans une productivité volumique faible, c'est-à-dire une concentration en micro-algues plus faible à l'issu de cette étape de culture, et une mauvaise maitrise des conditions de culture,
- les systèmes d'architecture de type fermée, connus sous l'appellation « photo-bioréacteurs », constitués par une ou plusieurs enceinte(s), c'est-à-dire dont l'intérieur n'est pas en contact avec l'air ambiant. Bien que nécessitant un investissement initial important, les photo-bioréacteurs permettent comparativement à ceux ouverts, d'atteindre de meilleures productivités volumiques et de réaliser des cultures dans des conditions mieux maitrisées, du fait notamment qu'on évite les contaminations, pollutions, et qu'on puisse injecter du CO₂ tout en limitant les pertes de ce CO₂ à l'atmosphère.

De manière générale, en dehors de quelques espèces cultivées pour des marchés de niche, les méthodes de culture des micro-algues n'ont pas encore atteint leur niveau de maturité industrielle.

Des problématiques de constance de qualité et de coût de production se posent encore, ce qui limite l'accès des micro-algues à de nombreux marchés commerciaux.

Enfin, la culture des micro-algues nécessite de grandes quantités d'eau et de nutriments, ce qui nuit grandement aux bilans environnementaux et énergétiques, limitant le développement de cette filière industrielle à des applications très spécifiques: [2].

En effet, la production mondiale de micro-algues se situe autour de 20000 tonnes à l'heure actuelle et s'adresse à des marchés de niche tels que la cosmétique, la nutraceutique ou encore le domaine médical : [3].

Cependant, le potentiel des micro-algues pour la chimie verte et l'énergie (biocarburants) est considérable: [4].

Ainsi, résoudre le problème des besoins en eau et en nutriments permettrait de gravir une étape importante vers la viabilité de la valorisation des micro-algues en chimie verte et en énergie.

La solution la plus prometteuse consiste à coupler la culture des micro-algues et le traitement d'effluents liquides d'origine urbaine, industrielle ou agricole, ce qui est connu depuis longtemps et a été initié dans les années 1960 en Californie [5]. Les effluents liquides contiennent généralement des polluants azotés et phosphorés qui peuvent être utilisés comme nutriments par les micro-algues: [6].

Les polluants organiques ou les micropolluants contenus dans ces effluents liquides peuvent amener des dysfonctionnements dans le traitement de ces effluents, car ils posent des problèmes de toxicité vis-à-vis des micro-algues et risquent de ralentir voire stopper leur croissance : [7], [8]. Les micro-algues sont capables de dégrader certaines de ces molécules organiques, mais les plus persistantes vont s'accumuler dans le milieu et donc poser des problèmes de toxicité.

Ces molécules, comme par exemple celles de la grande famille des micropolluants, peuvent facilement être captées par des charbons actifs qui sont largement utilisés dans le domaine du traitement des eaux pour la captation de micropolluants: [9], [10]. Des procédés industriels ont été réalisés sur la base de cette technique, par exemple, le procédé commercialisé sous la dénomination Actiflo® Carb de la société Veolia Environnement.

D'autre part, une des voies de valorisation énergétique des micro-algues qui est déjà bien appréhendé est le procédé de pyrolyse : [11], [12], [13], [14].

La pyrolyse de micro-algues produit une bio-huile valorisable en biocarburant, du gaz et un résidu carboné appelé char qui n'est généralement pas valorisé. La valorisation seule de la bio-huile en biocarburant ne permet pas d'atteindre des bilans énergétique et économique favorables.

Certaines tentatives de valorisation du char issu de la pyrolyse de micro-algues ont déjà eu lieu. Tout d'abord, le char issu de la pyrolyse de micro-algues possède en général une grande quantité d'azote qui le rend intéressant pour l'enrichissement des sols : [15]. Cependant, cette application n'a pas encore été démontrée que ce soit techniquement ou économiquement.

D'autres tentatives ont été recensées comme la gazéification du char en atmosphère de CO₂ : [16], ou encore la production de nanotubes de carbone comme divulgué dans la demande de brevet US 2011/0158893.

D'autres modes de production de char que la pyrolyse, ont mis en évidence l'utilisation avantageuse du char obtenu pour la captation de polluants, comme le bleu de méthylène : [17], ou les métaux lourds [18].

Il existe un besoin pour améliorer les procédés de production de micro-algues couplés au traitement d'effluents liquides, qui mettent en oeuvre une culture des micro-algues et une pyrolyse de la biomasse séchée obtenue à partir des micro-algues cultivées, notamment en vue d'éliminer les espèces toxiques présentes dans les effluents liquides qui constituent les milieux de culture des micro-algues.

Le but général de l'invention est de répondre au moins en partie à ce besoin.

Un but particulier est de proposer un procédé de production de micro-algues et de traitement d'eflluents liquides qui réponde au but général et qui soit en outre économiquement plus viable que les procédés existants.

### Exposé de l'invention

Pour ce faire, l'invention a pour objet un procédé de production de micro-algues et de traitement d'au moins un effluent liquide, comportant les étapes suivantes:
a/ culture de micro-algues dans une zone de culture dans laquelle on fait circuler le ou les effluents liquides;
b/ pyrolyse de la biomasse séchée obtenue à partir des micro-algues cultivées selon l'étape a/ ;
c/ mise en contact du char obtenu par la pyrolyse selon l'étape b/ avec le ou les effluents liquides.

Les effluents liquides visés par l'invention sont principalement ceux d'origine urbaine, industrielle ou agricole.

Ainsi, l'invention permet à la fois de :
- supprimer des molécules présentes dans les effluents liquides qui servent de milieu à la culture des micro-algues, ces molécules étant toxiques pour la croissance des de microorganismes photosynthétiques, telles que des souches axéniques, des consortiums de micro-algues, des consortiums bactériens artificiels ou naturel... ;
- valoriser le char issu de la pyrolyse de micro-algues séchées en rendant le procédé de traitement selon l'invention économiquement plus viable que ceux selon l'état de l'art;
- capter des micropolluants et/ou des polluants présents dans les effluents liquides, tels que les métaux lourds, les micropolluants organiques (dont pesticides, biocides, résidus médicamenteux, hydrocarbures aromatiques polycycliques), ..., et jusqu'à présent non traités par l'étape de culture usuelle des micro-algues.

En particulier, la valorisation du char issu de la pyrolyse pour capter des micropolluants permet un gain économique et environnemental important. En effet, en général, les micropolluants ne sont pas éliminés par des systèmes classiques de traitement des effluents des stations d'épuration de type boues activées: [19].

De plus, le sujet de la réduction voire l'élimination des taux de micropolluants dans les rejets dans l'environnement fait l'objet de nombreuses directives européennes (2000/60/EC, 2008/105/EC et 2011/0429) qui incitent les pouvoirs publics à développer des solutions telles que celles proposées par l'invention.

L'étape c/ peut être avantageusement réalisée simultanément à l'étape a/ de culture, au sein de la zone de culture.

En fonction des configurations, l'étape c/ peut être réalisée en amont, en parallèle ou en aval de l'étape a/ de culture.

La mise en contact peut être effectuée avantageusement dans un bassin ou dans un réacteur agité, un réacteur sous aération, un réacteur à lit fixe, ou un réacteur à lit fluidisé.

Selon une variante de réalisation, la mise en contact est effectuée en aval de l'étape de récolte/concentration des micro-algues.

Selon un mode de réalisation avantageux, au moins une partie du gaz obtenu par la pyrolyse selon l'étape b/, principalement constitué de dioxyde de carbone (CO₂), de monoxyde de carbone (CO), d'hydrogène (H₂) et d'hydrocarbures légers (CₙH₂ₙ) étant réinjectée dans la zone de culture.

Selon une variante de ce mode, au moins une partie du gaz réinjectée étant récupéré en sortie de la zone de culture puis brûlé afin de générer de la chaleur apte à être utilisée.

Selon une autre variante de ce mode, au moins une partie du gaz est réinjectée dans une colonne liquide montante sous dépression avec le milieu de culture, comprenant le ou les effluents liquides et les micro-algues, et permettant sa mise en circulation dans le réacteur de culture, et le gaz étant ainsi épuré de tout ou partie de son CO₂ et récupéré de manière séparée en sortie de la colonne. La colonne liquide sous dépression peut être avantageusement réalisée comme décrit dans la demande de brevet FR 2 952 370 A1. Une telle colonne est un système avantageux pour mettre en circulation le gaz issu de la pyrolyse et le milieu de culture composé des microalgues et des effluents, tout en permettant une économie d'énergie.

Selon une variante avantageuse, au moins une partie de l'huile obtenue par la pyrolyse selon l'étape b/ est brûlée dans une chaudière afin de générer de la chaleur apte à être utilisée.

Selon un mode de réalisation avantageux, le procédé comprend une cogénération de chaleur et d'électricité à partir de la combustion d'au moins une partie de l'huile et/ou du gaz obtenu(s) par la pyrolyse selon l'étape b/.

La chaleur et/ou l'électricité obtenues par la cogénération peu(ven)t être avantageusement utilisée(s) en amont dans le procédé.

Ainsi, plusieurs variantes avantageuses peuvent être envisagées :
- l'électricité générée par la cogénération peut être utilisée pour alimenter au moins une partie des équipements dédiés à la culture et/ou à la récolte/concentration des micro-algues cultivées et/ou du séchage des micro-algues récoltées/concentrées ;
- la chaleur générée par la cogénération peut être utilisée pour le séchage des micro-algues récoltées/concentrées ;
- l'électricité et/ou la chaleur générée(s) par la cogénération peu(ven)t être utilisée(s) pour alimenter le réacteur de pyrolyse pour la mise en oeuvre de l'étape b/ de pyrolyse.

Avantageusement, le char obtenu selon l'étape b/ de pyrolyse est brûlé dans l'unité de cogénération une fois la mise en contact selon l'étape c/ effectuée, afin de régénérer le char.

Les effluents liquides mis en circulation dans la zone de culture selon l'étape a/ et mise en contact avec le char selon l'étape c/, sont de préférence des effluents urbains, industriels ou agricoles.

### Description détaillée

D'autres avantages et caractéristiques de l'invention ressortiront mieux à la lecture de la description détaillée de l'invention faite à titre illustratif et non limitatif en référence aux figures suivantes parmi lesquelles :
- la figure 1 est une représentation schématique de l'enchaînement des étapes d'un procédé de traitement de micro-algues et d'effluents liquides d'algues selon un premier mode de l'invention ;
- la figure 2 est une représentation schématique de l'enchaînement des étapes d'un procédé de traitement de micro-algues et d'effluents liquides d'algues selon un deuxième mode de l'invention ;
- la figure 3 est une représentation schématique de l'enchaînement des étapes d'un procédé de traitement de micro-algues et d'effluents liquides d'algues selon un troisième mode de l'invention ;
- la figure 4 est une représentation schématique de l'enchaînement des étapes d'un procédé de traitement de micro-algues et d'effluents liquides d'algues selon un premier mode de l'invention ;
- la figure 5 est une représentation schématique de l'enchaînement des étapes d'un procédé de traitement de micro-algues et d'effluents liquides d'algues selon un cinquième mode de l'invention.

Dans la description qui va suivre les termes « entrée », « sortie » « amont », « aval », sont utilisés par référence avec la direction de circulation des effluents liquides, des micro-algues et du char dans le procédé de traitement selon l'invention.

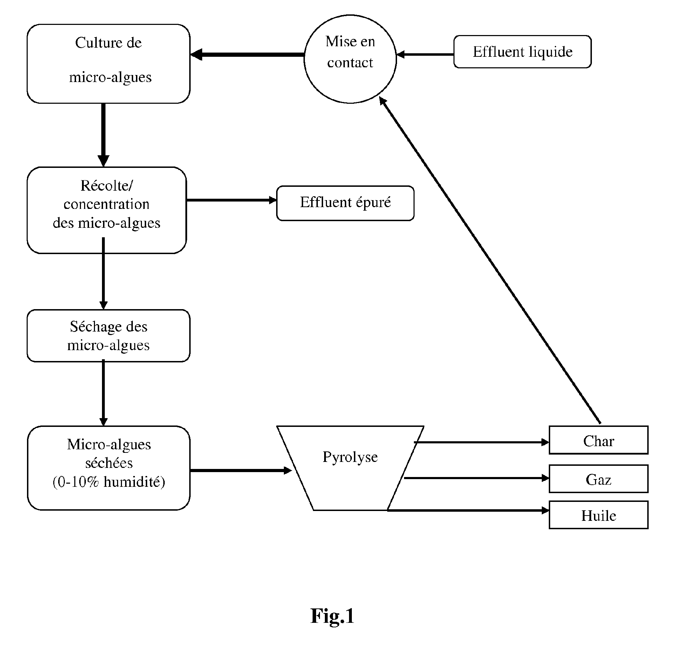

Comme représenté en figure 1, le procédé selon l'invention est mis en oeuvre en continu dans le traitement simultané d'effluents liquides et de micro-algues, avec les étapes successives suivantes.

On fait tout d'abord circuler un ou des effluent(s) liquide(s) dans une zone de culture dans laquelle des micro-algues peuvent être cultivées.

Au cours d'un processus de photosynthèse, le carbone minéral, sous la forme de dioxyde de carbone CO₂, dissous, est converti en carbone organique, constituant de la matière organique des micro-algues. Cette croissance dite photo-autotrophe permet aux micro-algues de se diviser très efficacement, avec un taux de doublement de 8h en conditions optimales.

Lorsqu'une source de carbone organique est disponible comme un sucre simple, un acide organique, certaines micro-algues peuvent croître de manière hétérotrophe en assimilant cette forme de carbone.

La zone de culture des micro-algues peut être un système d'architecture de type ouverte, également connus sous la dénomination « Raceway », lorsque ce type de système est optimisé (agitation par roues à aubes et forme ovale particulière), qui est constitués par des bassins ou lagunes à ciel ouvert, c'est-à-dire à l'air libre. Cela peut être également un système d'architecture fermée de type photo-bioréacteur intensifs.

Les lagunes sont des systèmes simples, efficaces et très communément utilisés pour la culture des micro-algues : [4]. Il existe différents designs depuis la lagune naturelle jusqu'au « Raceways » optimisés. Dans ces derniers, il est mis en oeuvre une agitation à l'aide d'une ou plusieurs roue(s) à aube, une géométrie en forme d'anneau permettant une optimisation de l'écoulement. Parfois, les « Raceways » peuvent comprendre un système d'aération permettant l'adjonction de CO₂, en tant que source de carbone pour les micro-algues.

La profondeur des bassins est généralement comprise entre 20 et 50 cm ce qui limite d'autant le transfert de lumière, le fond des bassins ne recevant généralement pas ou très peu de lumière. Ceci explique la relativement faible productivité volumique qui peut être atteinte par ces systèmes, typiquement égale à 0,1 g de biomasse/litre/jour.

Une autre problématique rencontrée par les systèmes ouverts concerne l'exposition aux contaminations. En effet, des microorganismes (protozoaires, rotifères, ...) peuvent facilement contaminer les bassins par voie aérienne. Dans le traitement de l'invention où des effluents liquides sont traités, il y a formation d'un consortium microbien incluant en majorité des micro-algues, mais également de nombreux autres microorganismes. Du fait du traitement d'effluents liquide et de la pyrolyse réalisée sur les micro-algues, la présence de ces autres microorganismes n'est pas problématique pour la mise en oeuvre de l'invention.

Enfin, l'évaporation dans les bassins permet de garder des températures raisonnables dans les cultures mais elle implique une consommation en eau élevée.

Les photo-bioréacteurs (PBR) sont quant à eux des réacteurs clos spécifiquement conçus pour la culture de microorganismes photosynthétiques, tels que les micro-algues. Leur principale caractéristique est que leurs parois en verre ou en polymère sont transparentes, afin de laisser passer la lumière nécessaire à la croissance des micro-algues. Les PBR les plus couramment utilisés comprennent des tubes horizontaux, des colonnes verticales ou de fines plaques verticales.

Une fois, la culture des micro-algues réalisée, on procède à l'étape de leur récolte/concentration.

La concentration en micro-algues dans le milieu de culture est généralement faible, de l'ordre de 0,5 à 5 g/L, selon le type de zone de culture choisi, i.e. raceways ou photo-bioréacteurs. En raison de leur petite taille, les micro-algues se caractérisent par une stabilité colloïdale. Un colloïde est un mélange d'un liquide et d'une suspension de particules de si petites tailles qu'elles se répartissent de façon homogène.

Le milieu de culture selon l'invention ne contient pas uniquement des micro-algues, mais cela ne gêne pas l'étape de récolte ni celle de séchage en aval. Certaines bactéries, notamment filamenteuses, peuvent par ailleurs favoriser la floculation et donc la concentration en flocs des microorganismes, facilitant ainsi leur récolte.

Les différentes techniques de récolte de la biomasse algale peuvent être envisagées dans le cadre de l'invention.

En premier, la coagulation-floculation, souvent appelée par le seul terme floculation, est une technique éprouvée dans le domaine du traitement des eaux usées et consiste en l'augmentation de la taille des particules par agrégation des cellules. Ella a été décrite comme la plus prometteuse puisqu'elle permet de traiter de grandes quantités de ces particules en suspension ainsi qu'une large variété de micro-algues: [20]. Elle est de surcroit économiquement viable, bien que plusieurs techniques de floculation se distinguent, dont certaines sont encore au stade de recherche et développement et donc plus onéreuses à mettre en oeuvre. La forme, la taille et la composition des flocs divergent fortement selon l'espèce de micro-algues et le floculant utilisé : [21]. En revanche, un floculant idéal serait bon marché, non toxique, efficace à faible concentration et devrait dériver de préférence d'une source d'énergie non fossile, être renouvelable et biodégradable: [22].

On distingue plusieurs méthodes pour coaguler/floculer une suspension de micro-algues que l'on peut énumérer comme suit :
- l'auto-floculation s'opère souvent de manière spontanée lorsque le pH du milieu de culture est supérieur à 9: [23]. Un pH élevé induit une formation de précipités inorganiques qui peuvent être chargés positivement, lesquels interagissent avec la surface des cellules algales et conduisent à une floculation : [24]. De manière analogue, un pH inférieur ou égal à 5 favorise une coagulation des cellules, même si ces dernières peuvent rester en suspension dans le milieu de culture et sédimenter au bout de quelques minutes : [25] ;
- l'électrofloculation qui consiste à induire une floculation au moyen d'une libération d'ions métalliques de l'anode sacrificielle : [26] et/ou d'imposer des pulsations électromagnétiques pour neutraliser la charge surfacique des cellules : [27].
- la biofloculation qui consiste à ajouter à la culture algale des bactéries et autres micro-organismes qui, par leur charge positive peuvent induire une floculation: [28]. Dans le traitement selon l'invention, la présence naturelle d'autres microorganismes peut faciliter la floculation ;
- la floculation chimique qui consiste à ajouter dans le milieu de culture, des sels minéraux ou des polymères organiques naturels ou synthétiques, ce qui permet une floculation relativement efficace qui s'opère en deux étapes. La première consiste en la neutralisation des charges négatives entourant les colloïdes, suivie par une interaction des particules entre elles à travers des liaisons ioniques ou hydrogène.

Ces techniques de floculation-coagulation sont très intéressantes d'un point de vue économique, énergétique et technique, cependant, elles ne permettent pas d'atteindre des facteurs de concentration suffisant et constituent généralement une étape de pré-concentration en amont d'une étape de concentration que peut être la filtration ou la centrifugation.

Les techniques de filtration sont également des techniques de récolte qui peuvent être mise en oeuvre dans le procédé de traitement selon l'invention.

Tout d'abord, la filtration sur membranes est un procédé dont la mise en oeuvre est facile, et dont le principe repose sur une exclusion par la taille. Elle peut être frontale, avec l'écoulement perpendiculaire à la toile ou au tamis ou bien tangentielle avec dans ce cas le fluide qui entre en contact parallèlement à la surface du filtre, permettant ainsi de s'affranchir partiellement des contraintes d'obstruction précoces. Si les membranes mises en oeuvre présentent des pores de l'ordre de 0,5 à 0,02 µm, on parle de micro et d'ultrafiltration.

Seulement, les coûts des membranes, ainsi que la consommation énergétique élevée (afin de réaliser une différence de pression suffisante pour permettre la filtration) rendent cette technologie non adaptée pour une valorisation énergétique de la biomasse.

La filtration peut ainsi être considérée comme une technique intéressante pour la récolte des micro-algues dans le traitement selon l'invention, mais il faut envisager des développements pour le passage à l'échelle industrielle du fait du niveau de maturité insuffisant à ce jour.

La technologie des filtres à bandes pressantes, qui provient du domaine du traitement des eaux est prometteuse pour le traitement des micro-algues selon l'invention puisqu'elle permet des gains économiques et énergétiques par rapport aux procédés classiques par centrifugation : [2].

En ce qui concerne cette dernière, elle consiste à extraire les micro-algues sont de leur milieu de culture grâce à la variation de l'intensité gravitationnelle imposée par un mouvement de rotation très rapide. Les caractéristiques des particules solides à séparer (taille, concentration...), le temps de séjour ou encore l'accélération en sont les paramètres opératoires clés.

La centrifugation est à l'heure actuelle la méthode la plus utilisée pour la récolte des micro-algues : [22], [29]. C'est en effet la méthode la plus pratique à mettre en oeuvre pour des volumes importants et c'est donc tout naturellement la solution préférentiellement utilisée par les industriels du secteur. La centrifugation permet d'atteindre des concentrations de l'ordre de 12 à 22% de matières sèches dans le concentrat: [30], la viabilité cellulaire est comprise entre 88 et 100% et le taux de récupération >95% : [31].

Cependant, la contrainte majeure de la récolte des micro-algues par centrifugation reste son importante consommation énergétique, comprise entre 4 et 12 kWh/kg de biomasse, ce qui la rend incompatible avec une exploitation industrielle à visée énergétique : [2].

Seule la technologie de décanteurs centrifuges semble convenir à la mise en oeuvre de l'invention. En effet, les décanteurs centrifuges sont plus adaptés pour traiter des suspensions plus riches en solides. C'est la raison pour laquelle, des procédés en amont sont préconisés pour augmenter la concentration en micro-algues des suspensions à traiter afin d'optimiser l'aspect économique.

Une fois que les micro-algues sont récoltées, on réalise leur séchage qui est nécessaire du fait de la conversion par pyrolyse que l'on souhaite leu faire subir. En effet, la pyrolyse nécessite une biomasse à très faible teneur en eau, typiquement au maximum de 10%).

Deux types de sécheurs peuvent être envisagés dans le traitement selon l'invention :

- des sécheurs thermiques de type à tambour rotatif ou séchoir-convoyeur qui permettent d'éliminer l'eau résiduelle par évaporation. Avec ce type de sécheurs, on peut atteindre des taux d'humidité inférieur à 5% dans la biomasse sèche ;

- des sécheurs qui utilisent l'énergie solaire, qui ont pour avantage majeur d'améliorer le bilan énergétique. Ces sécheurs sont soit des systèmes sous serre généralement utilisés pour le séchage de boues issues du traitement d'effluents liquides, soit des sécheurs spécifiquement dédiés.

Lorsque le séchage de la biomasse algale est réalisé, on peut procéder à son traitement par pyrolyse.

Le procédé de pyrolyse est une réaction qui a lieu dans un réacteur de pyrolyse (lit fixe, lit fluidisé, réacteur à flux entrainé, ...) en absence d'oxygène, à une température comprise entre 400 et 700°C, et qui produit trois catégories de produits:
- des condensables, regroupés communément sous le terme d'huile,
- du gaz, mélange d'H₂, CO, CO₂, CH₄ et d'hydrocarbures légers et,
- un résidu solide appelé char.

Ces produits obtenus (huile, gaz et char) sont séparés : le gaz est récupéré dans la partie haute du réacteur de pyrolyse, l'huile et le char sont séparés par filtration, décantation ou centrifugation.

L'huile qui est le produit principal de la pyrolyse peut être valorisée énergétiquement soit directement dans une chaudière ou une unité de cogénération, soit être raffinée en biocarburant liquide. Cependant, les bilans énergétique et économique ne sont pas favorables lorsque seule l'huile de pyrolyse est valorisée. Une étude récente indique même qu'avec la valorisation seule de l'huile, les bilans énergétiques sont négatifs et le coût de production est de l'ordre de 2,5€/litre de biocarburant: [32].

Cette valorisation rend jusqu'à ce jour le procédé de traitement des micro-algues non viable économiquement.

Comme évoqué en préambule, il a déjà été imaginé de valoriser le char comme amendement pour les sols ou en le brûlant dans une chaudière afin de générer de la chaleur.

Les inventeurs ont pensé à une autre voie de valorisation du char qui consiste à l'utiliser afin d'adsorber et capter les micropolluants dans les effluents liquides qui servent de support à la culture des micro-algues par apport d'eau et de nutriments tels que l'azote et phosphore.

Autrement dit, l'étape de mise en contact entre char issu de la pyrolyse et les effluents liquides selon l'invention permet tout d'abord de d'adsorber et capter les micropolluants qui sont toxiques pour la croissance des micro-algues, ce qui permet une meilleure productivité en biomasse.

La valorisation du char selon l'invention apporte donc une valeur économique et environnementale supplémentaire par rapport aux procédés de traitement selon l'état de l'art.

On précise ici que la mise en contact peut être faite par tout moyen qui permette d'amener le char obtenu dans une zone de mélange avec les effluents liquides contact optimum.

Ainsi, la mise en contact entre le char et l'effluent liquide peut se faire dans un bassin ou dans un réacteur dédié, agité ou sous aération ou encore dans un lit fixe ou un lit fluidisé (figures 1, 3, 4 et 5).

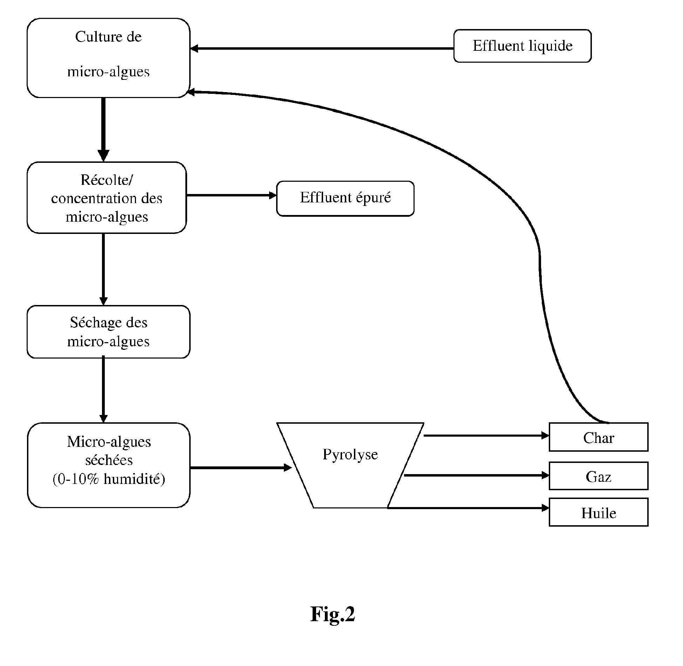

Alternativement, le char peut être directement ajouté dans la zone de culture (figure 2).

Différents modes de mise en contact entre char et effluents liquides peuvent être envisagés dans le cas où la zone de culture est un bassin ou un réacteur dédié.

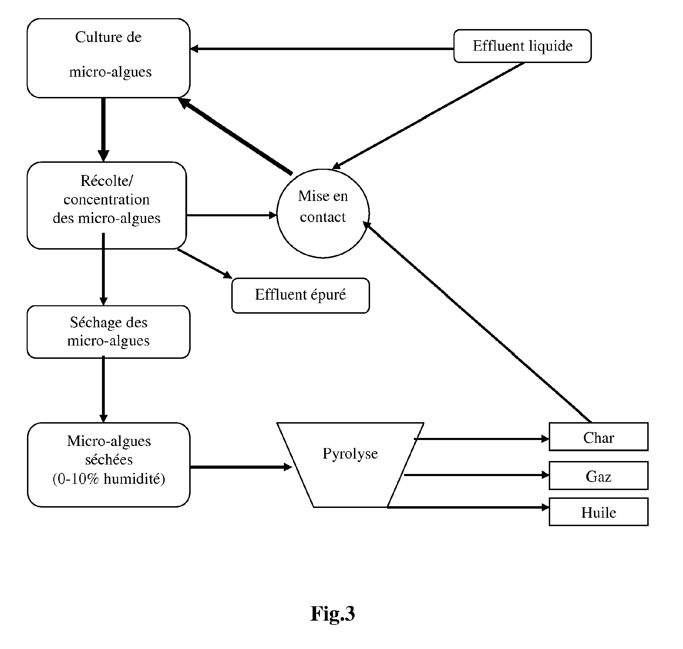

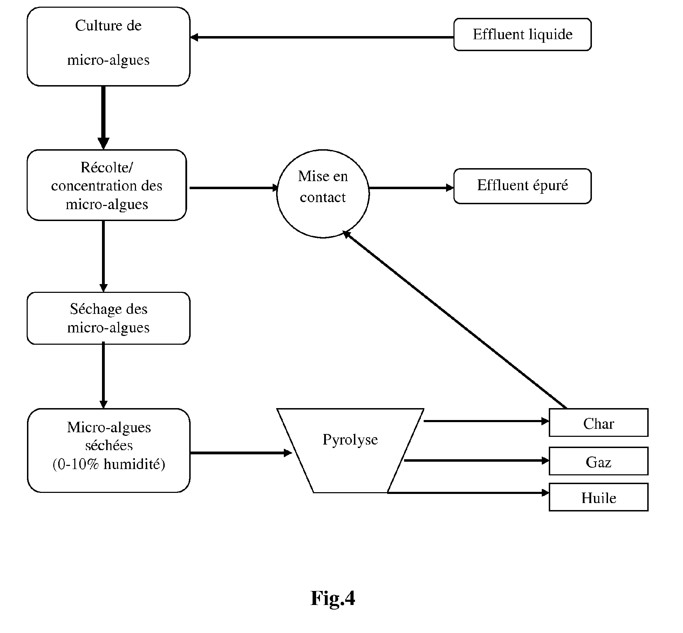

La mise en contact peut ainsi s'effectuer :
- en amont de l'étape de culture (figure 1) ;
- en parallèle de l'étape de culture (figure 3) ;
- voire en aval dans le cas où il n'y aurait pas de composés toxiques pour l'étape de culture dans les effluents utilisés (figure 4).

Selon une variante, on peut envisager une étape supplémentaire de raffinage de l'huile obtenue par pyrolyse si on souhaite la transformer en biocarburant. Ainsi l'huile issue de la pyrolyse des micro-algues contient de l'oxygène, environ 10% en masse, et des traces d'azote et de soufre. Ces composés peuvent être substitués par des atomes d'hydrogène dans le procédé d'hydrotraitement. Après séparation des produits par distillation, on obtient différentes fractions dont du biodiesel et du bio-kérosène.

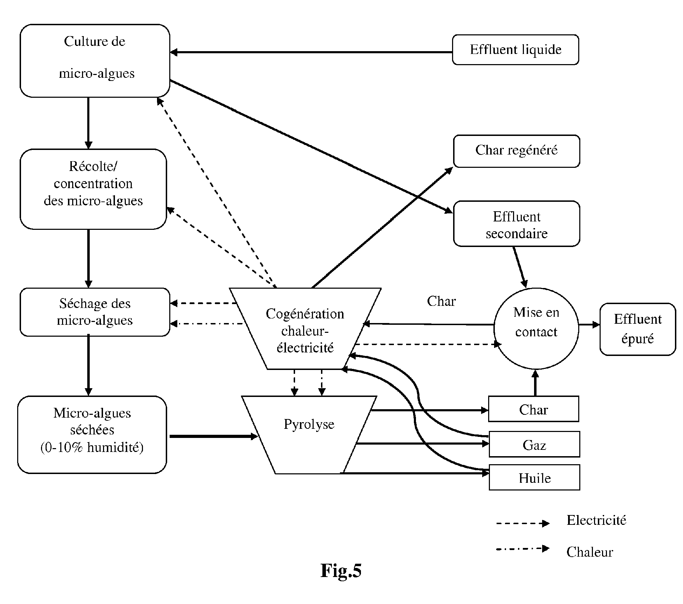

Selon un mode de réalisation avantageux, on peut prévoir d'intégrer à l'installation mettant en oeuvre le procédé de traitement selon l'invention, une unité de cogénération pour la production combinée de chaleur et d'électricité, comme schématisé en figure 5.

Selon ce mode, le gaz produit par pyrolyse, qui possède un pouvoir calorifique non négligeable, peut être valorisé dans l'unité de cogénération.

Il en va de même avec au moins une partie de l'huile de pyrolyse. En effet, dans le cas où l'huile issue de la pyrolyse des micro-algues n'est pas transformée en biocarburant, elle peut être brûlée dans l'unité de cogénération afin de produire chaleur et électricité.

La chaleur et l'électricité ainsi produites par l'unité de cogénération peuvent ainsi avantageusement être utilisées dans l'ensemble des étapes du procédé de traitement selon l'invention :
- l'électricité peut servir à alimenter électriquement les équipements dédiés respectivement à la culture des micro-algues, à leur récolte, à leur séchage et au réacteur de pyrolyse ;
- la chaleur peut être fournie aux équipements de séchage et/ou au réacteur de pyrolyse.

Dans ce mode avec intégration d'une unité de cogénération, le char usagé sur lequel les polluants ont été adsorbés et captés peut être brûlé dans l'unité.

D'autres variantes et améliorations peuvent être prévues sans pour autant sortir du cadre de l'invention.

L'invention n'est pas limitée aux exemples qui viennent d'être décrits ; on peut notamment combiner entre elles des caractéristiques des exemples illustrés au sein de variantes non illustrées.

### Références citées

[1] : J. Pruvost et Al. «production industrielle de microalgues et cyanobactéries », Techniques de l'ingénieur, IN 200, 11/2011
[2] : Delrue, F., Setier, P-A., Sahut, C., Cournac, L., Roubaud, A., Peltier, G., Froment, AK. 2012. "An economic, sustainability, and energetic model of biodiesel production from microalgae". Bioresour Technol 111:191-200;
[3] : Spolaore, P., Joannis-Coassan, C., Duran, E., Isambert, A. 2006. "Commercial applications of microalgae". Journal of Bioscience and Bioengineering 101, 87-96;
[4] : Brennan, L. and Owende, P. 2010. "Biofuels from microalgae-a review of technologies for production, processing, and extractions of biofuels and co-products". Renew. Sust. Energy Rev. 14, 557-577;
[5] : Oswald, W.J. 1963. "High rate ponds in waste disposal". Development of Industrial Microbiology 4, 112-119.
[6] : Zhou, W., Chen, P., Min, M., Ma, X., Wang, J., Griffith, R., Hussain, F., Peng, P. Xie, Q., Li, Y., Shi, J., Meng, J., Ruan, R. 2014. "Environment-enhancing algal biofuel production using wastewaters". Renewable and Sustainable Energy Reviews 36, 256-269;
[7] : Munoz, R., Guieysse, B. 2006. "Algal-bacterial processes for the treatment of hazardous contaminants: A review". Water Research 40, 2799-2815;
[8] : Kumar, K.S., Dahms, H.U., Lee, J-S, Kim, H.C., Lee, W.C., Shin, K-H. 2014. "Algal photosynthetic responses to toxic metals and herbicides assessed by chlorophyll a fluorescence". Ecotoxicology and Environmental Safety 104, 51-71.
[9] : Quinlivan PA, Li L, Knappe DRU, 2005. "Effects of activated carbon characteristics on the simultaneous adsorption of aqueous organic micro-pollutants and natural organic matter". Water Research 39, pp. 1663-1673;
[10] : Reungoat J, Macov M, Escher BI, Carswell S, Mueller JF, Keller J. 2010. "Removal of micropolluants and redutction of biological activity in a full scale reclamation plant using ozonation and activated carbon filtration". Water Research 44, pp. 625-637;
[11] : Miao X, Wu Q. 2004. "High yield bio-oil production from fast pyrolysis by metabolic controlling of Chlorella protothecoides". J Biotechnol 110, pp. 85-93;
[12] : Miao X, Wu Q, Yang C. 2004. "Fast pyrolysis of microalgae to produce renewable fuels ". J Anal Appl Pyrolysis 71, pp. 855-63;
[13] : Gong X, Zhang B, Zhang Y, Huang Y, Xu M. 2014. « Investigation on pyrolysis of low lipid microalgae chlorella vulgaris and dunaliella salina". Energy Fuels 28, pp. 95-103;
[14] : Belotti G, De Caprariis B, De Filippis P, Scarsella M, Verdone N. 2014. « Effect of Chlorella vulgaris growing conditions on bio-oil production via fast pyrolysis ». Biomass and Bioenergy 61, pp. 187-95 ;
[15] : Grierson S, Strezov V, Bengtsson J. 2013. "Life cycle assessment of a microalgae biomass cultivation, bio-oil extraction and pyrolysis processing regime". Algal Research 2, pp. 299-311;
[16] : Yang C, Jiao L, Su S, Tian Z, Song Q, Fang W, Chen C, Liu G. 2012. "Utilization of CO2 and biomass char derived from pyrolysis of Dunaliella salina: The effects of steam and catalyst on CO and H2 gas production". Bioresource Technology 110, pp 676-681;
[17] : Leng L, Yuan X, Huang H, Wang H, Wu Z, Fu L, Peng X, Chen X, Zeng G. 2015. "Characterization and application of bio-chars from liquefaction of microalgae, lignocellulosic biomass and sewage sludge". Fuel Processing Technology 129, pp 8-14;
[18] : Inyang M, Gao B, Yao Y, Xue Y, Zimmerman AR, Pullammanappallil P, Cao X. 2012. "Removal of heavy metals from aqueous solution by biochars derived from anaerobically digested biomass". Bioresource Technology 110, pp 50-56;
[19] : Choubert, J. M., Martin Ruel, S., Esperanza, M. Budzinski, H., Miège, C., Lagarrigue, C. et Coquery, M. 2010. "Limiting the emissions of micro-pollutants: what efficiency can we expect from wastewater treatment plants? Water Science & Technology 63, pp. 57-65 ;
[20] : Uduman, N et al. 2010. "Dewatering of microalgal cultures: a major bottleneck to algae-based fuels''.J. Renew. Sust. Energy 2, 012701;
[21] : Jago, C.F., Kennaway, G.M., Novarino, G., Jones, S.E. 2007. "Size and settling velocity of suspended flocs during a Phaeocystis bloom in the tidally stirred Irish Sea, NW European shelf"'. Marine Ecol Prog Ser 345:51-62;
[22] : Molina-Grima, E., Belarbi, E.H., Acien Fernandez, F.G., Robles Medina, A., Chisti, Y. 2003. "Recovery of microalgal biomass and metabolites: process options and economics". Biotechnology Advances 20, 7-8, pp. 491-515;
[23] : Spilling, K. et al. 2011. "Inducing autofloculation in the diatom Phaeodactylum tricornutum through CO2 régulation". J. Appl. Phycol. 23, 959-966;
[24] : Vandamme, D. et al. 2012. "Floculation of Chlorella vulgaris induced by high pH: role of magnesium and calcium and practical implications". Bioresour. Technol. 105, 114-119;
[25] : Liu J., Zhu Y., Tao Y., Zhang Y., Li A., Li T., Sang M., Zhang C. 2013. « Freshwater microalgae harvested via floccatiation indliced by pH decrease". Biotechnology for Biofuels 6:98;
[26] : Vandamme, D. et al. 2011. "Evalatation of electro-coagulation-flocculation for harvesting marine and freshwater- microalgae". Biotechnol Bioeng. 108, 2320-2329;
[27] : Gouveia, L. 2011. "Microalgae as Feedstock for Biofuels". Springer;
[28] : Zang, J., Hu, B. 2012. "A novel method to harvest microalgae via co-culture of filamentous fungi to form cell pellets". Bioresour. Technol. 114, 529-535;
[29]: Mohn, F. 1980. "Experiences and strategies in the recovery of biomass from mass cultures of microalgae". Algae Biomass, pp. 545-571;
[30]: Logan Christenson, Ronald Sims. "production and harvesting of microalgae for wastewater treatment, biofuels, and bioproducts". Biotechnology Advances, 2011 ;
[31]: Heasman, M., Diemar, J., O' Connor, W., Sushames, T., Foulkes, L., Nell, J.A. 2000. "Development of extended shelf-life microalgae concentrate diets harvested by centrifugation for bivalve molluscs-a summary". Aquacult. Res., 31 (8-9), pp. 637-659;
[32]: Hognon et al, 2015

## Revendications

1. Procédé de production de micro-algues et de traitement d'au moins un effluent liquide, comportant les étapes suivantes:
a/ culture de micro-algues dans une zone de culture dans laquelle on fait circuler le ou les effluents liquides;
b/ pyrolyse de la biomasse séchée obtenue à partir des micro-algues cultivées selon l'étape a/ ;
c/ mise en contact du char obtenu par la pyrolyse selon l'étape b/ avec le ou les effluents liquides.

2. Procédé de traitement selon la revendication 1, l'étape c/ étant réalisée simultanément à l'étape a/ de culture, au sein de la zone de culture.

3. Procédé de traitement selon la revendication 1, l'étape c/ étant réalisée en amont, en parallèle ou en aval de l'étape a/ de culture.

4. Procédé de traitement selon la revendication 3, la mise en contact étant effectuée dans un bassin ou dans un réacteur agité, un réacteur sous aération, un réacteur à lit fixe, ou un réacteur à lit fluidisé.

5. Procédé de traitement selon l'une des revendications 3 ou 4, la mise en contact étant effectuée en aval de l'étape de récolte/concentration des micro-algues.

6. Procédé de traitement selon l'une des revendications précédentes, au moins une partie du gaz obtenu par la pyrolyse selon l'étape b/, principalement constitué de dioxyde de carbone (CO₂), de monoxyde de carbone (CO), d'hydrogène (H₂) et d'hydrocarbures légers (CₙH₂ₙ) étant réinjectée dans la zone de culture.

7. Procédé de traitement selon la revendication 6, au moins une partie du gaz réinjectée étant récupéré en sortie de la zone de culture puis brûlé afin de générer de la chaleur apte à être utilisée.

8. Procédé de traitement selon la revendication 6 ou 7, au moins une partie du gaz étant réinjectée dans une colonne liquide montante sous dépression avec le milieu de culture, comprenant le ou les effluents liquides et les micro-algues, et permettant sa mise en circulation dans le réacteur de culture, et le gaz étant ainsi épuré de tout ou partie de son CO₂ et récupéré de manière séparée en sortie de la colonne.

9. Procédé de traitement selon l'une des revendications précédentes, au moins une partie de l'huile obtenue par la pyrolyse selon l'étape b/ étant brûlée dans une chaudière afin de générer de la chaleur apte à être utilisée.

10. Procédé de traitement selon l'une des revendications précédentes, comprenant une cogénération de chaleur et d'électricité à partir de la combustion d'au moins une partie de l'huile et/ou du gaz obtenu(s) par la pyrolyse selon l'étape b/.

11. Procédé de traitement selon la revendication 10, l'électricité générée par la cogénération étant utilisée pour alimenter au moins une partie des équipements dédiés à la culture et/ou à la récolte/concentration des micro-algues cultivées et/ou du séchage des micro-algues récoltées/concentrées.

12. Procédé de traitement selon la revendication 10 ou 11, la chaleur générée par la cogénération étant utilisée pour le séchage des micro-algues récoltées/concentrées.

13. Procédé de traitement selon l'une des revendications 10 à 12, l'électricité et/ou la chaleur générée(s) par la cogénération étant utilisée(s) pour alimenter le réacteur de pyrolyse pour la mise en oeuvre de l'étape b/ de pyrolyse.

14. Procédé de traitement selon l'une des revendications 10 à 13, le char obtenu selon l'étape b/ de pyrolyse étant brûlé dans l'unité de cogénération une fois la mise en contact selon l'étape c/ effectuée, afin de régénérer le char.

15. Procédé de traitement selon l'une des revendications précédentes, les effluents liquides mis en circulation dans la zone de culture selon l'étape a/ et mise en contact avec le char selon l'étape c/, étant des effluents urbains, effluents industriels ou agricoles.
